# EUROPÄISCHE PATENTANMELDUNG

(11) **EP 1 955 732 A1**
(43) Veröffentlichungstag der Anmeldung: **13.08.2008**
(21) Anmeldenummer: 07023807.6
(22) Anmeldetag: 08.12.2007
(51) Int. Cl.: A61N 1/375, A61N 1/378, H01R 13/24

(54) **Kontaktmodul für ein medizinisches Implantat**

(30) Priorität: 07.02.2007 DE 102007006088
(71) Anmelder: BIOTRONIK CRM Patent AG, 6341 Baar (CH)
(72) Erfinder: Starke, Marcel, 12167 Berlin (DE)
(74) Vertreter: Lindner-Vogt, Karin L.

(57) **Zusammenfassung**

Die Erfindung betrifft eine elektrische Verbindung zwischen den einzelnen Komponenten aktiver medizinischer Implantate, insbesondere zwischen einer Spannungsquelle und einem elektrischen Verbraucher. Dabei ist die Spannungsquelle mit dem elektrischen Verbraucher über einen Federkontaktblock elektrisch leitend verbunden. Die elektrische Verbindung zwischen der Spannungsquelle und dem elektrischen Verbraucher ist dabei durch den Federkontaktblock hergestellt, indem die Kontaktierfläche des Federkontaktes auf einer Gegenkontaktfläche des Verbrauchers oder der Spannungsquelle aufliegt und eine durch die Feder-Rückstellkraft des Federkontaktes erzeugte Anpresskraft auf die Gegenkontaktfläche ausübt, wobei die Gegenkontaktfläche im Wesentlichen eben ausgebildet ist und parallel zu der Oberfläche, auf der sie befestigt ist, angeordnet ist.

## Beschreibung

Die Erfindung betrifft eine elektrische Verbindung zwischen den einzelnen Komponenten aktiver medizinischer Implantate, insbesondere zwischen einer Spannungsquelle und einem elektrischen Verbraucher.

Aktive medizinische Implantate, vor allem medizinische Impulsgeneratoren, Herzschrittmacher oder Kardioverter-Defibrillatoren sind Systeme, die eine äußerst niedrige Ausfallwahrscheinlichkeit bzw. Fehlerrate aufweisen müssen, da sie für die Gesundheit eines Patienten eine wichtige, teilweise lebenswichtige, Aufgabe erfüllen. Aus diesem Grund werden bei der Herstellung medizinischer Impulsgeneratoren besonders weitreichende Qualitätssicherungsmaßnahmen durchgeführt.

Neben der ständigen Durchführung von Qualitätskontrollen während des Herstellungsprozesses werden bei Impulsgeneratoren auch besonders hochwertige elektronische Bausteine und Materialien verwendet.

Aktive medizinische Implantate, auf welche sich die vorliegende Patentanmeldung bezieht, besitzen üblicherweise ein Gehäuse, in dem elektrische Komponenten des lmplantats untergebracht sind. Hauptkomponenten sind eine Spannungsquelle, also z.B. eine Batterie sowie elektrische Verbraucher, die von der Spannungsquelle mit Energie zu versorgen sind. Die elektrischen Verbraucher sind üblicherweise in Form von Schaltungen aufgebaut, die z.B. eine Leiterplatte und darauf montierte elektrische und elektronische Bauteile, wie z.B. integrierte Schaltungen, aufweisen. Die Schaltungen bilden z.B. Impulsgeneratoren zum Erzeugen elektrischer Stimulationsimpulse, die an eine Kammer eines Herzens abgegeben werden können, um eine Kontraktion der jeweiligen Kammer zu stimulieren.

Um den fehlerfreien Betrieb von lmpulsgeneratoren auch nach der Implantation zumindest für die geplante Betriebsdauer zu gewährleisten, sind diese von einem zumindest flüssigkeitsdichten, in der Regel aber auch hermetisch dichtem Gehäuse umgeben, welches das Eindringen von Blut und Gewebsflüssigkeit in das Innere des Gehäuses, verhindert.

Das Gehäuse besteht z.B. aus Titan, und ist üblicherweise aus zwei Halbschalen zusammengesetzt, die nach Fertigstellung des Implantats dicht miteinander verbunden sind, so dass das Gehäuse anschließend geschlossen ist.

Einmal geschlossen, sind die Gehäuse nur noch mit Gewalt zu öffnen, und es ist nicht vorgesehen, einzelne Komponenten, wie z. B. die Spannungsquelle, nach einer gewissen Zeit auszutauschen. Vielmehr sind implantierbare Herzschrittmacher, Kardioverter und Defibrillatoren zur einmaligen Verwendung vorgesehen und werden nach Ablauf der zulässigen Betriebszeit durch ein neues Gerät ersetzt. Dazu ist eine Explantation des alten Gerätes und die Implantation eines neuen Gerätes notwendig.

Weil ein Austausch von Systemkomponenten bei solchen Implantaten nicht vorgesehen und aufgrund von sicherheitstechnischen Überlegungen sogar gesetzlich untersagt ist, werden die einzelnen Komponenten der Implantate gemäß dem Stand der Technik, insbesondere die Komponenten Spannungsquelle und elektrischer Verbraucher, aber auch andere Komponenten, durch feste Verbindungen elektrisch leitend miteinander verbunden.

Typischerweise werden feste, elektrisch leitende Verbindungen zwischen einzelnen Komponenten oder Leitungen durch Löten oder Schweißen, selten durch Crimpen oder eine andere Art kraft- oder formschlüssiger Verbindungen hergestellt.

Da sich die intrakardiale Herztherapie mittlerweile zu einem weltweit millionenfach bewährtem Standardverfahren entwickelt hat, ist es aus Kostengründen sinnvoll, das Herstellungsverfahren der Implantate zu automatisieren. Der Aufbau gängiger elektromedizinischer Implantate lässt sich dabei vereinfacht wie folgt beschreiben. Bisher werden alle Komponenten, wie eine Batterie, ein Schaltkreis, evtl. eine Telemetrieeinheit oder dergleichen, meist nebeneinander im Implantatsgehäuse untergebracht. Das Implantatsgehäuse selber hat in der Regel eine flache, langgestreckte Kontur mit abgerundeten Rändern und wird in der Regel aus zwei Halbschalen mit einer Art Schnappmechanismus aus ineinandergreifenden Kanten gebildet.

Eine solche Anordnung, bei der die einzelnen Komponenten meist durch eine feste Verbindung, insbesondere durch Löten miteinander elektrisch verbunden sind, hat allerdings den Nachteil, dass bei der Montage der einzelnen Komponenten mehrere Fertigungsachsen angesteuert werden müssen. Dies erschwert eine Automatisierung und führt zu erhöhten Kosten.

Die elektrische Verbindung zwischen energie-verbrauchenden Komponenten und der Batterie muss außerdem auch folgender Problematik Rechnung tragen: Während der Entladung der Batterie kommt es in Folge der zugrundeliegenden elektrochemischen Reaktion zu einer geringen Volumenzunahme der Batterie. Dieses entladungsbedingte Anschwellen der Batterie kann bei der elektrischen Verbindung zwischen der Batterie und dem Schaltkreis, oder einer anderen, mit der Batterie verbundenen Komponente, zu Problemen führen.

Aufgabe der vorliegenden Erfindung ist es, den Aufbau des Implantats unter dem Gesichtspunkt eines möglichst einfachen und automatisierbaren Herstellungsverfahrens zu optimieren, ohne dass dadurch die Zuverlässigkeit des Implantats beeinträchtigt ist.

Diese Aufgabe wird erfindungsgemäß durch ein aktives medizinisches Implantat gelöst, welches über ein Gehäuse verfügt, das zumindest zwei elektrische Komponenten, z.B. eine Batterie und eine Schaltung, flüssigkeitsdicht umschließt. Das Implantat zeichnet sich dadurch aus, dass
- wenigstens zwei elektrische Komponenten über einen Federkontaktblock elektrisch leitend verbunden miteinander sind,
- wobei wenigstens eine der elektrisch leitenden Komponenten wenigstens eine Gegenkontaktfläche aufweist, die als flaches, elektrisch leitendes Oberflächenelement einer Oberfläche der Komponente ausgebildet ist,
- wobei der Federkontaktblock wenigstens einen elektrisch isolierenden Kontaktträger und wenigstens einen von diesem gehaltenen, leitfähigen, elastischen Federkontakt aufweist,
- der wenigstens zwei elektrisch leitende Kontaktierflächen aufweist, die elektrisch leitend miteinander verbunden sind, und
- der so ausgebildet ist, dass wenigstens eine der Kontaktierflächen unter Druck auf die Kontaktierflächen elastisch federnd ausgelenkt wird und eine definierte Feder-Rückstellkraft aufbaut,
- wobei eine elektrische Verbindung zwischen zwei elektrisch leitenden Komponenten durch den Federkontaktblock so hergestellt ist, dass die Kontaktierfläche des Federkontaktes auf wenigstens einer Gegenkontaktfläche wenigstens einer Komponente aufliegt und eine durch die Feder-Rückstellkraft des Federkontaktes erzeugte Anpresskraft auf die Gegenkontaktfläche ausübt.

Während der Entladung einer Batterie kommt es, wie bereits weiter oben beschrieben, zu einer geringen Volumenzunahme der Batterie. Dies kann bei herkömmlichen Impulsgeneratoren zu Problemen führen. Ein Vorteil der vorliegenden Erfindung besteht darin, dass ein Anschwellen der Batterie dabei mit Hilfe der Federkontakte des Federkontaktblocks besonders gut ausgeglichen werden kann, da eine Relativbewegung zwischen dem Schaltkreis und der Batterie möglich ist.

Deshalb kann der Kontaktträger bevorzugt einen mechanischen Anschlag bilden, der Relativbewegungen senkrecht oder waagerecht zur oberen Fläche des Kontaktträgers verhindert. Dadurch werden mechanische Belastungen, die entweder durch die Volumenvergrößerung der Batterie oder äußere Belastung im Gebrauch und bei der Herstellung entstehen können, auf die relativ stabile Stromquelle, die auch bevorzugt eine Batterie ist, abgeführt, was Beschädigungen des Verbrauchers und seiner Komponenten verhindert.

Ein weiterer Vorteil der vorliegenden Erfindung besteht darin, den Fertigungsprozess von medizinischen Impulsgeneratoren dadurch zu vereinfachen, dass herkömmliche, feste elektrische Verbindungen, insbesondere Lötverbindungen zwischen einzelnen Komponenten, vermieden werden.

Stattdessen kommt eine elektrische Verbindung zwischen den elektrisch leitenden Komponenten des erfindungsgemäßen Implantats beim Zusammenfügen der jeweiligen Komponenten automatisch durch die Feder-Rückstellkraft der Kontaktfedern zwischen den Kontaktierflächen der Federkontakte und den Gegenkontaktflächen auf der entsprechenden Komponente zustande.

Der Federkontaktblock verfügt gemäß einer besonders bevorzugten Ausführungsvariante der vorliegenden Erfindung über mehrere Federkontakte, die einen im Wesentlichen flachen Querschnitt aufweisen.

Hierbei weisen die Federkontakte vorzugsweise wenigstens eine Biegung auf, welche ausschließlich in einer Ebene verläuft, die senkrecht zu einer Oberfläche des Federkontaktes, vorzugsweise der Flachseite verläuft. Bevorzugt weist die Biegung einen Radius von weniger als 2 mm auf.

Die Kontaktierflächen der Federkontakte sind gemäß einer besonders bevorzugten Ausführungsform konvex in Richtung der Gegenkontaktflächen gewölbt.

Vorzugsweise sind die Federkontakte aus kupferhaltigen Legierungen hergestellt und besitzen eine Kontaktierfläche, die aus einer gold- oder nickelhaltigen Legierung besteht. Dabei besitzen die Federkontakte bevorzugt einen flachen Querschnitt von weniger als 1 mm und werden in einem gebogenen Zustand von dem Kontaktträger gehalten.

Vorzugsweise verfügt die elektrische Komponente, welche die Gegenkontaktflächen aufweist, über eine den Federkontakten des Federkontaktblocks entsprechende Anzahl von Gegenkontaktflächen. Die Gegenkontaktflächen sind vorzugsweise flache Elemente, die entweder direkt in eine Oberfläche der entsprechenden elektrische Komponente eingelassen sind, oder sie werden von einem Gegenkontaktträger gehalten, welcher seinerseits in eine Oberfläche der entsprechenden Komponente eingelassen ist.

Gemäß einer besonders bevorzugten Ausführungsvariante sind die Kontaktierflächen der Federkontakte und/ oder die Gegenkontaktflächen mit einer gold- oder nickelhaltigen Legierung beschichtet, um eine Vergrößerung des elektrischen Widerstandes infolge von Korrosion zu verhindern.

Gemäß einer besonders bevorzugten Ausführungsvariante sind der elektrische Verbraucher und die Spannungsquelle über den Federkontaktblock elektrisch leitend miteinander verbunden.

Besonders bevorzugt ist dabei der Federkontaktblock ein oberflächenmontierbares Bauteil und ist direkt auf die Leiterplatte gelötet und so elektrisch leitend mit dieser verbunden.

Gemäß dieser bevorzugten Ausführungsvariante, verfügt die Batterie über wenigstens eine Gegenkontaktfläche.

Das medizinische Implantat ist vorzugsweise so aufgebaut, dass das Gehäuse des Implantats von zwei Halbschalen gebildet ist, welche am Ende des Herstellungsprozesses zusammengefügt und verschweißt werden. Dadurch sind die in dem Gehäuse befindlichen Komponenten des Implantats flüssigkeitsdicht umschlossen.

Vorzugsweise ist dabei die Spannungsquelle in einer der beiden Halbschalen untergebracht, und der elektrische Verbraucher in der jeweils anderen Halbschale. Vorzugsweise sind sowohl die Spannungsquelle, als auch der elektrische Verbraucher fest mit der jeweiligen Halbschale verbunden.

Gemäß einer bevorzugten Ausgestaltung der Erfindung, besteht das Implantatgehäuse aus zwei Halbschalen, wobei eine der Halbschalen gleichzeitig Bestandteil des Batteriegehäuses ist.

Sind gemäß der besonders bevorzugten Ausführungsvariante die Spannungsquelle und der elektrische Verbraucher über den Federkontaktblock elektrisch leiten miteinander verbunden, so sind der Federkontaktblock und die Gegenkontaktflächen innerhalb der jeweiligen Halbschalen so angeordnet, dass die Federkontakte durch das Zusammenfügen der beiden Halbschalen, von den Gegenkontaktflächen vorgespannt werden. Die daraus resultierende Feder-Rückstellkraft bewirkt, dass die Kontaktierflächen der Federkontakte auf die Gegenkontaktflächen gedrückt werden, und eine elektrische Verbindung zwischen der Spannungsquelle und dem elektrischen Verbraucher hergestellt ist.

Neben der bislang vorgeschlagenen, besonders bevorzugten Anordnung des Federkontaktblocks und der Gegenkontaktflächen, entsprechen zahlreiche weitere Anordnungen von Federkontaktblock und Gegenkontaktflächen unterschiedlichen Ausführungsvarianten der vorliegenden Erfindung.

Gemäß einer dieser Ausführungsvariante verfügt sowohl der elektrische Verbraucher, als auch die Spannungsquelle über Gegenkontaktflächen, die so angeordnet sind, dass sie die Federkontakte des Federkontaktblocks bei dem Zusammenfügen der beiden Halbschalen eindrücken. Dadurch sind sowohl die Gegenkontaktflächen des elektrischen Verbrauchers, als auch die Gegenkontaktflächen der Batterie über die Kontaktierflächen der Federkontakte mit dem Federkontaktblock elektrisch leitend verbunden. Dadurch ist eine elektrische Verbindung zwischen der Spannungsquelle und dem elektrischen Verbraucher hergestellt.

Wenn eine elektrische Verbindung gemäß dieser Ausführungsvariante zustande kommt, ist der Federkontaktblock mit keiner der Komponenten fest verbunden. Um eine horizontale Verschiebung des Federkontaktblocks zu verhindern, was dazu führen könnte, dass die Kontaktierflächen der Federkontakte nicht mehr auf den Gegenkontaktflächen aufliegen, weist gemäß einer bevorzugten Ausführungsvariante zumindest eine der beiden Halbschalen oder eine der beiden elektrische Komponenten eine Halterung auf, die eine entsprechende Bewegung des Federkontaktblocks verhindert.

Gemäß einer weiteren Ausführungsvariante ist der Federkontaktblock als oberflächenmontierbares Bauteil fest mit der Batterie und elektrisch leitend mit den Polen der Batterie verbunden. In diesem Fall verfügt vorzugsweise der elektrische Verbraucher über Gegenkontaktflächen. Die Gegenkontaktflächen sind dabei so angeordnet, dass sie bei dem Zusammenfügen der beiden Halbschalen die Federkontakte des Federkontaktblocks eindrücken, wodurch eine elektrische Verbindung zwischen der Batterie und dem elektrischen Verbraucher hergestellt ist.

Gemäß weiterer Ausführungsvarianten stellen ein oder mehrere Federkontaktblöcke auch elektrische Verbindungen zwischen mehreren elektrischen Komponenten des elektrischen Verbrauchers her. Auf diese Weise könne medizinische Implantate sandwichartig ohne Löten, Schweißen oder dergleichen besonders einfach zusammengesetzt werden.

Ein weiterer Aspekt der vorliegenden Erfindung betrifft ein Verfahren zur Herstellung des weiter oben beschriebenen medizinischen Implantats. In diesem Zusammenhang werden im Folgenden einige besonders vorteilhafte Verfahrensschritte zur Herstellung des oben genannten Gegenstandes der vorliegenden Erfindung genannt.

Demnach bildet die Spannungsquelle, welche durch Kleben fest mit einer der Halbschalen verbunden ist, die Basis für die Montage. In der anderen Halbschale befindet sich, vorzugsweise fest mit dieser verbunden, der elektrische Verbraucher mit wenigstens einem Schaltkreis auf einer Leiterplatte. Bevorzugt ist der Federkontaktblock bereits als oberflächenmontierbares Bauteil auf der Leiterplatte aufgelötet.

In einem der folgenden Verfahrensschritte werden beide Halbschalen zusammengefügt. Dabei werden die Federkontakte des Federkontaktblocks eingedrückt und vorgespannt. Die daraus resultierende Feder-Rückstellkraft drückt die Kontaktierflächen der Federkontakte auf die Gegenkontaktflächen der Spannungsquelle, wodurch eine elektrische Verbindung hergestellt ist.

Besonders bevorzugt werden diese Verfahrensschritte für gebördelte Gehäuse eingesetzt. Nach dem Zusammenfügen der Halbschalen, bei dem die elektrische Verbindung zwischen der elektrischen Spannungsquelle und dem Schaltkreis automatisch hergestellt worden ist, werden die beiden Halbschalen vorzugsweise luft- und flüssigkeitsdicht versiegelt.

Im Folgenden werden einige besonders bevorzugte Ausführungsvarianten der vorliegenden Erfindung in Verbindung mit den Figuren 1 bis 9 erläutert.
- Figur 1: ist eine Draufsicht auf die beiden Gehäusehälften des Implantats in einem geöffneten Zustand.
- Figur 2: ist eine perspektivische Darstellung der elektrisch leitenden Gegenkontakt-flächen.
- Figur 3: ist eine perspektivische Darstellung des Federkontaktblocks.
- Figur 4: ist eine Querschnittsdarstellung der elektrischen Verbindung zwischen Spannungsquelle und elektrischem Verbraucher.
- Figur 5: ist eine Querschnittsdarstellung des medizinischen Implantats gemäß der Hauptausführungsvariante, mit einem als oberflächenmontierbares Element auf der Leiterplatte befestigtem Federkontaktblock.
- Figur 6: ist eine Querschnittsdarstellung einer zweiten Ausführungsvariante der vorliegenden Erfindung .
- Figur 7: ist eine Querschnittsdarstellung gemäß einer dritten Ausführungsvariante der vorliegenden Erfindung.
- Figur 8: ist eine Querschnittsdarstellung gemäß einer vierten Ausführungsvariante der vorliegenden Erfindung.
- Figur 9: ist eine Querschnittsdarstellung gemäß einer fünften Ausführungsvariante der vorliegenden Erfindung.

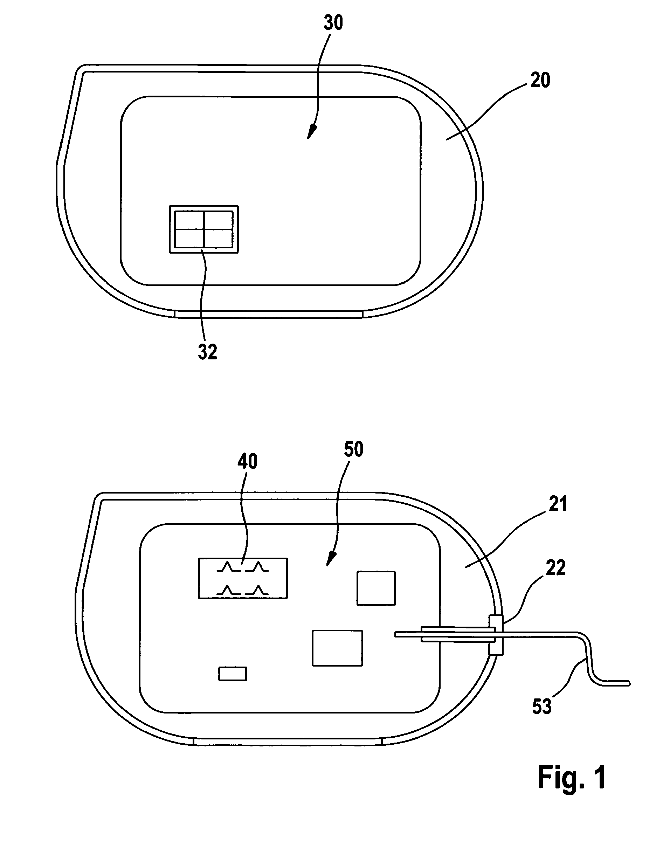

Figur 1 zeigt die Grundbausteine eines aktiven medizinischen Implantats. Das Implantat umfasst zwei Gehäusehälften, die als Halbschalen 20 und 21 ausgebildet sind. In der ersten Halbschale 20 ist eine Batterie 30 als Spannungsquelle untergebracht und vorzugsweise fest mit der Halbschale 20 durch zum Beispiel Kleben verbunden.

Die zweite Halbschale umfasst einen elektrischen Verbraucher 50.

Um bei dem Zusammenfügen der beiden Halbschalen, im Rahmen eines Herstellungsverfahrens für das Implantat, eine elektrische Verbindung zwischen Batterie 30 und Verbraucher 50 herzustellen, umfasst die Batterie gemäß dieser Ausführungsvariante vier Gegenkontaktflächen, die als flache, elektrisch leitende Oberflächenelemente ausgebildet sind und welche elektrisch leitend mit der Batterie 30 verbunden sind.

Der elektrische Verbraucher 50 verfügt über einen Federkontaktblock 40, welcher seinerseits mit dem elektrischen Verbraucher 50 elektrisch leitend verbunden ist.

Wenn die beiden Halbschalen in einem späteren Herstellungsschritt zusammengefügt werden, wird dadurch auch die elektrische Verbindung zwischen dem elektrischen Verbraucher und der Batterie hergestellt, wie in den nachfolgenden Figuren detaillierter beschrieben. Der Verbraucher wird dadurch in die Lage versetzt, elektrische Signale zu verarbeiten und über einen Leiterstrang 53, welcher durch eine Durchführung 22 der Halbschale 21 geführt ist, mit einem zu behandelnden oder zu überwachenden Ort auszutauschen, bzw. zu übertragen.

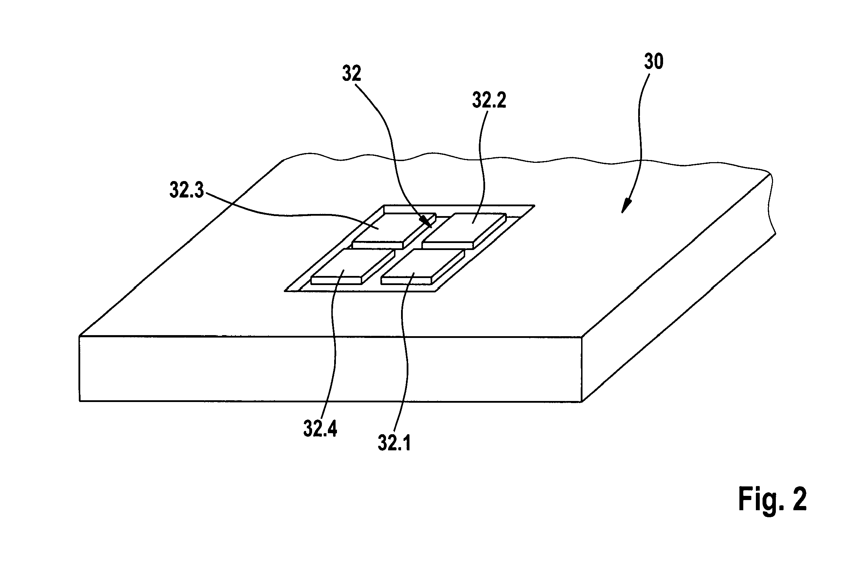

Figur 2 zeigt einen Ausschnitt der Batterie 30 mit einem Gegenkontaktträger 32. In dem Gegenkontaktträger 32, sind gemäß dieser Ausführungsform vier Gegenkontaktflächen 32.1, 32.2, 32.3 und 32.4 eingebettet. Diese Gegenkontaktflächen 32.1, 32.2, 32.3 und 32.4 sind mit den Polen der Batterie 30 elektrisch leitend verbunden. Jede der Gegenkontaktflächen 32.1, 32.2, 32.3 und 32.4 bildet dabei ein flaches, elektrisch leitendes Oberflächenelement der Batterie.

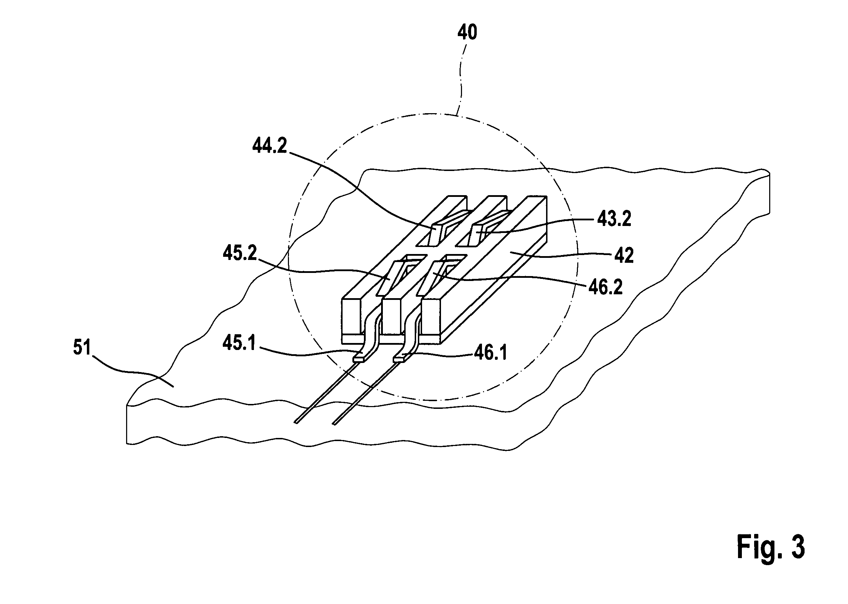

Figur 3 zeigt eine perspektivische Teilansicht des Verbrauchers 50, auf dem der Federkontaktblock 40 aufgebracht ist. Bei dem Federkontaktblock 40 handelt es sich vorzugsweise um ein oberflächenmontierbares Bauteil, das direkt auf eine Leiterplatte 51 des elektrischen Verbrauchers 50, aufgelötet ist. Dabei umfasst der Federkontaktblock 40 einen elektrisch isolierenden Kontaktträger 42, in welchem gemäß dieser Ausführungsvariante vier Federkontakte 43, 44, 45 und 46 gehalten sind. Dabei weist jeder der Federkontakte wenigstens zwei leitende Kontaktierflächen 43.1, 43.2, 44.1, 44.2, 45.1, 45.2, 46.1 und 46.2 auf. Jeweils eine Kontaktierfläche 43.1, 44.1, 45.1 und 46.1 jedes Federkontaktes ist gemäß dieser Ausführungsvariante elektrisch leitend mit der Leiterplatte 51 verbunden. Die zweite Kontaktierfläche 43.2, 44.2, 45.2 und 46.2 jedes Federkontaktes, welche elektrisch leitend mit jeweils der ersten Kontaktierfläche verbunden ist, ragt nach oben aus dem Kontaktträger 42 heraus.

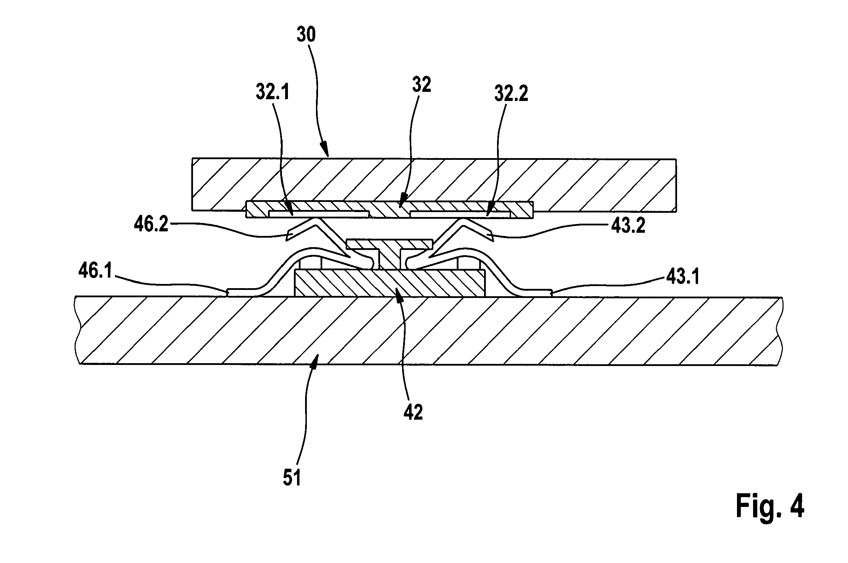

Figur 4 ist eine Querschnittsdarstellung der elektrischen Verbindung zwischen Spannungsquelle und elektrischem Verbraucher. Von unten nach oben zeigt die Darstellung den Kontaktträger 42, der auf der Leiterplatte 51 aufgebracht ist. Der Kontaktträger 42 hält die beiden Federkontakte 45 und 46. Diese weisen, wie bereits weiter oben beschrieben, jeweils zwei Kontaktierflächen 45.1, 45.2 bzw. 46.1 und 46.2 auf, welche jeweils elektrisch leitend miteinander verbunden sind. An den Kontaktierflächen 45.1 und 46.1 sind die Federkontakte elektrisch leitend mit der Leiterplatte 51 verbunden und sind bevorzugt auf dieser aufgelötet. Oberhalb der Kontaktierflächen 45.2 und 46. 2 befinden sich elektrisch leitend mit den Kontaktierflächen verbunden die beiden flach ausgebildeten Gegenkontaktflächen 32.1 und 32.2. Diese stehen in elektrischer Verbindung mit den Polen der Batterie 30. Dabei bilden die Gegenkontaktflächen 32.1 und 32.2 jeweils zwei elektrisch leitende Oberflächenelemente der Batterie 30. Bevorzugt - wie in dieser Figur dargestellt - sind die beiden Gegenkontaktflächen in einem Gegenkontaktträger 32 eingefasst, welcher seinerseits in der Oberfläche der Batterie 30 eingefasst und dadurch fest mit dieser verbunden ist.

Die elektrische Verbindung zwischen den Kontaktierflächen und den Gegenkontaktflächen kommt dadurch zustande, dass beim Zusammenfügen der beiden Halbschalen 20 und 21, die beiden Gegenkontaktflächen 32.1 und 32.2 eine Kraft auf die Kontaktierflächen 45.2 und 46.2 ausüben. Dadurch werden die Federkontakte 45 und 46 vorgespannt und stellen mit Hilfe der dadurch erzeugten Feder-Rückstellkraft eine dauerhafte elektrische Verbindung zwischen den Kontaktierflächen 45.2, 46.2 und den Gegenkontaktflächen 32.1 und 32.2 her. Dadurch ist die Versorgung der Leiterplatte 51 und damit auch insgesamt des elektrischen Verbrauchers 50 durch die Batterie 30 sichergestellt.

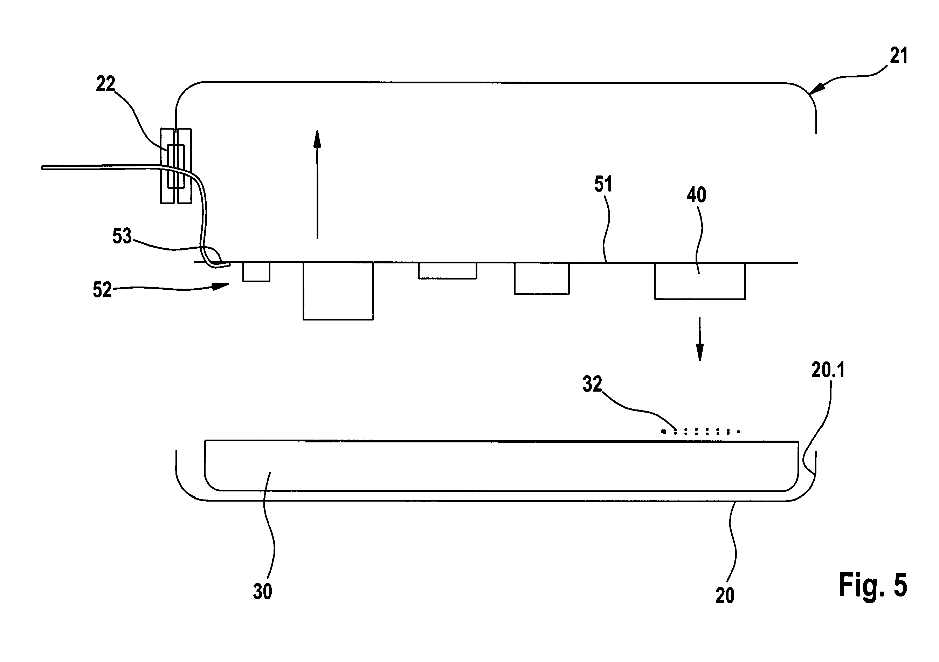

Figur 5 zeigt die Querschnittsdarstellung einer bevorzugten Ausführungsvariante des medizinischen Implantats, welches über die Halbschalen 20 und 21 verfügt. Die beiden Halbschalen 20, 21 sind dabei als Schnappschalen mit ineinandergreifenden Kanten ausgebildet.

Halbschale 20, die in einem besonders bevorzugten Herstellungsverfahren des medizinischen Implantats als Basis dient, umfasst die Batterie 30 als Spannungsquelle, welche mit der Halbschale 20 durch Kleben stoffschlüssig verbunden ist. Dabei ist zwischen der umlaufenden Schmalseite der Batterie 30 und der Halbschale 20 vorzugsweise ein kleiner Abstand 20.1 vorgesehen, der der Batterie Raum für eine während des Entladevorgangs auftretende Expansion gibt. In der in Bezug auf das Implantat nach innen gewandten Oberfläche der Batterie 30 ist der Gegenkontaktträger 32 befestigt, in den die Gegenkontaktflächen eingebettet sind. Der Gegenkontaktträger 32 ist als oberflächenmontierbares Bauteil fest mit den Polen der Batterie 30 elektrisch leitend verbunden.

Neben der Batterie, als die erste elektrische Komponente des Implantats, ist ein auf einer Leiterplatte 51 montierter integrierter Schaltkreis als zweite elektrische Komponente des Implantats in der Halbschale 21 untergebracht. Dort sind gegebenenfalls weitere Komponenten des aktiven medizinischen Implantats untergebracht. Bevorzugt ist die Leiterplatte 51 fest mit der Gehäusehälfte 21 verbunden. Auf der Leiterplatte 51, die den integrierten Schaltkreis beinhaltet, sind entsprechend dieser Ausführungsvariante weitere oberflächenmontierbare Bauteile 52 aufgelötet. Der in der Leiterplatte 51 integrierte Schaltkreis und, falls vorhanden, alle weiteren funktionellen Komponenten, die in elektrischer Verbindung mit der Batterie 30 stehen, werden in dieser Patentanmeldung gemeinsam als elektrische Verbraucher 50 bezeichnet.

Wie in der Querschnittsdarstellung in Figur 5 erkennbar, ist der Federkontaktblock 40 als oberflächenmontierbares Bauteil so auf die Leiterplatte 51 aufgelötet, dass sich der Federkontaktblock 40 direkt über dem Gegenkontaktträger 32 befindet. Das erste Ende des Leiterstrangs 53 ist gemäß dieser Ausführungsvariante elektrisch leitend mit der Leiterplatte 51 verbunden. Der Leiterstrang 53 ist durch die Durchführung 22 des Gehäuses 21 geführt, so dass elektrische Signale von der Leiterplatte 51 zum zweiten Ende des Leiterstrangs 53, welches sich außerhalb des Gehäuses 21 befindet, geleitet werden können.

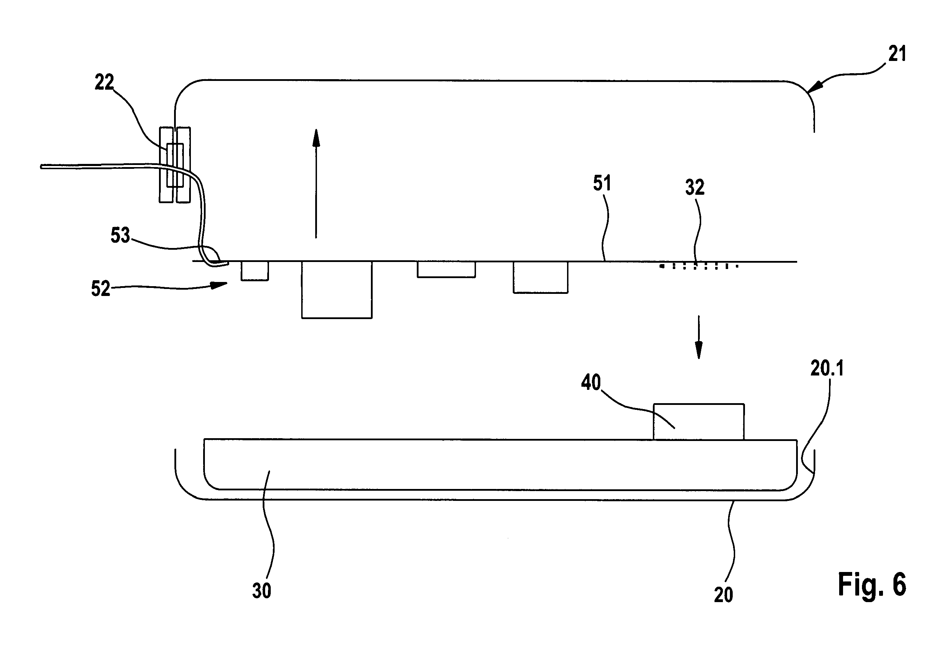

In der Fig. 6 ist schematisch eine weitere alternative Anordnung mit einem einachsigen Komponentenaufbau dargestellt. In den Grundzügen entspricht sie der bereits in der Fig. 5 vorgestellten Anordnung von Leiterplatte 51 und Batterie 30. Im Unterschied zu der Ausführungsform gemäß Figur 5, ist der Gegenkontaktträger 32, in dem die Gegenkontaktflächen eingebettet sind, fest mit der Leiterplatte 51 verbunden, und der Federkontaktblock ist fest mit der Batterie 30 und elektrisch leitend den Polen der Batterie 30 verbunden.

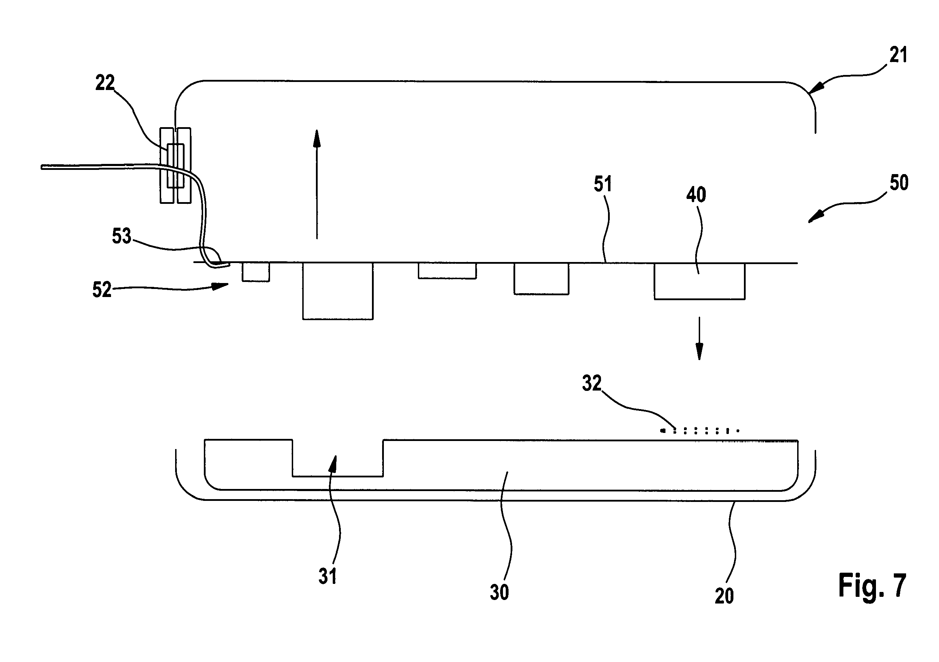

Figur 7 zeigt eine weitere Ausgestaltungsvariante des Gegenstandes der vorliegenden Patentanmeldung. Dabei entspricht die Anordnung von Leiterplatte 51 und Batterie 30, sowie von Federkontaktblock 40 und Gegenkontaktträger 32 der Ausführungsvariante aus Figur 5. Im Unterschied zu Figur 5 verfügt die Batterie 30 gemäß dieser Ausgestaltung über zumindest eine Aussparung 31, die es ermöglicht, dass die elektrischen Komponenten 52, die auf der Leiterplatte 51 aufgelötete sind und in vertikaler Richtung besonders große Abmessungen aufweisen, teilweise in der oder den Aussparungen 31 der Batterie 30 Platz finden und so eine kompaktere Bauweise des Implantats ermöglichen.

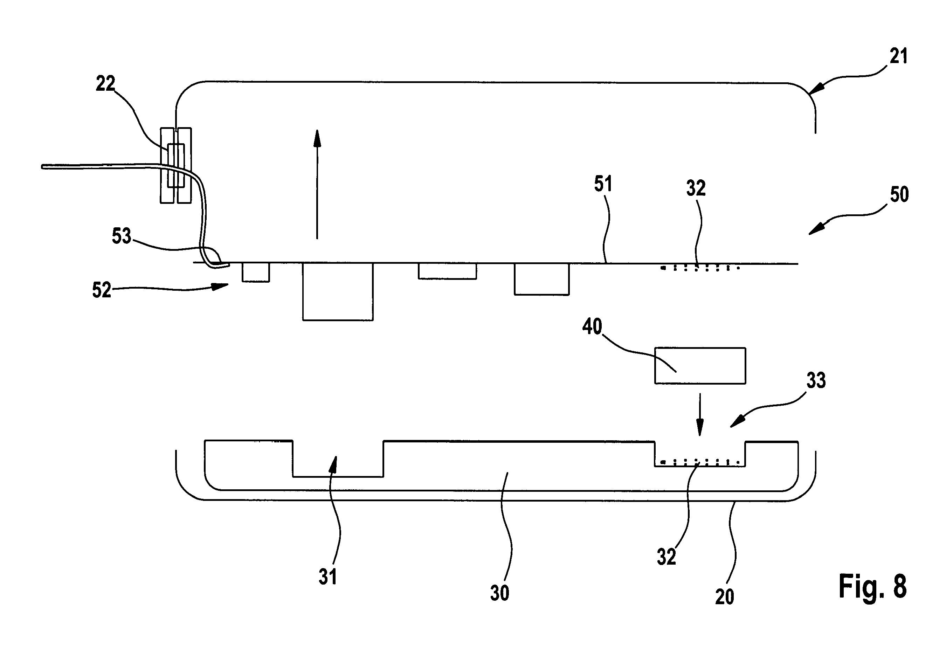

Figur 8 zeigt eine weitere Ausgestaltungsvariante eines aktiven Implantats. Im Gegensatz zu den in den Figuren 5 bis 7 dargestellten Ausführungsvarianten verfügt das Implantat bei dieser Ausgestaltung über zwei Gegenkontaktträger 32 und 34. Der Gegenkontaktträger 34 ist dabei fest mit der Leiterplatte verbunden. Der Gegenkontaktträger 32 befindet sich am Boden einer Aussparung 32 in der Batterie 30 und ist fest mit der Batterie verbunden. Der Federkontaktblock 40 ist gemäß dieser Ausführungsvariante kein oberflächenmontierbares Bauteil bzw. ist bei dieser Ausgestaltung weder mit der Leiterplatte 51 noch mit der Batterie 30 fest verbunden. Vielmehr wird der Federkontaktblock während der Herstellungsverfahrens des Implantats in die Aussparung 33 der Batterie 30 eingelegt.

Werden nun während eines späteren Herstellungsschrittes die Gegenkontaktflächen der Leiterplatte, welche eingebettet im Gegenkontaktträger 34 eingefasst sind, durch das Zusammenfügen der beiden Halbschalen auf die Kontaktierflächen der Federkontakte gedrückt, werden die Federkontakte vorgespannt. Die daraus resultierende Feder-Rückstellkraft bewirkt dadurch eine elektrische Verbindung zwischen den ersten Kontaktierflächen der Federkontakte und den Gegenkontaktflächen der Leiterplatte, sowie den zweiten Kontaktierflächen der Federkontakte und den Gegenkontaktflächen der Batterie, welche von Gegenkontaktträger 32 eingefasst sind.

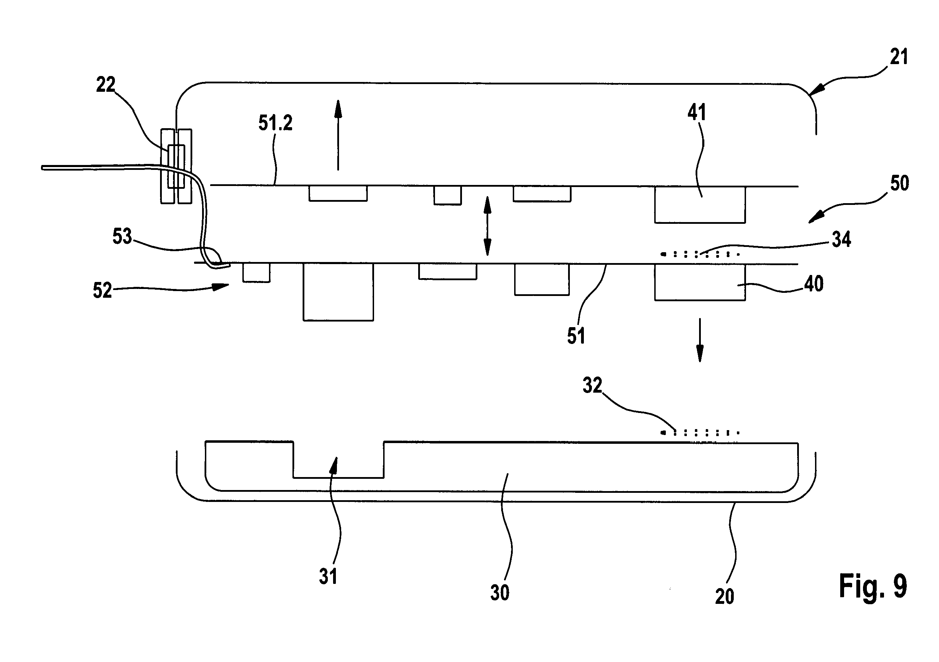

Figur 9 zeigt eine weitere Ausführungsform eines aktiven Implantats. Demnach können nach dem immer gleichen Prinzip einer mit Hilfe von Federkontaktblock und Gegenkontaktflächen hergestellten elektrischen Verbindung auch weitere Module von elektrischen Verbrauchern direkt oder indirekt über die Leiterplatte 51 mit den Polen der Batterie 30 verbunden werden. Gemäß der hier gezeigten Ausgestaltung ist auf der Batterie abgewandten Seite der Leiterplatte 51 ein Gegenkontaktträger 34 aufgebracht und elektrisch leitend mit der Leiterplatte 51 verbunden. Oberhalb der Leiterplatte 51 befindet sich Leiterplatte 51.2 als eine weitere funktionelle Gruppe des medizinischen Implantats. Auf der Leiterplatte 51 zugewandten Seite der Leiterplatte 51.2 ist der Federkontaktblock 41 fest und elektrisch leitend mit der Leiterplatte 51.2 verbunden. Das Zusammenfügen der Halbschalen bewirkt nun sowohl bei den Federkontakten des Federkontaktblocks 40, als auch bei den Federkontakten des Federkontaktblocks 41 eine Feder-Rückstellkraft, durch die sowohl zwischen Batterie und Leiterplatte 51, als auch zwischen Leiterplatte 51 und Leiterplatte 51.2 eine elektrische Verbindung hergestellt wird.

Bei allen der genannten Ausführungsvarianten weist der Gegenkontakt 40 einen isolierenden Kontaktträger 42 auf, der einerseits zur Isolation zwischen den Federkontakten 43, 44, 45 und 46 dient, andererseits auch den Federkontakten eine Führung senkrecht zur oberen Fläche des Kontaktträgers 42 bietet und die Federkontakte positioniert. Weiterhin kann der Grundkörper auch als mechanischer Anschlag, der zur Oberfläche des Kontaktträgers 42 senkrechte und/oder waagerechte Relativbewegungen zwischen den Komponenten - also beispielsweise der Spannungsquelle 30 und den Leiterplatten 51 und/oder 51.2 - verhindert. Diese Relativbewegungen resultieren beispielsweise aus der Schwellung der Batterie oder aber bei Freiheitsgraden im Innenaufbau. Zur Verhinderung dieser Relativbewegungen kann der Kontaktträger zum Beispiel eine definierte Höhe aufweisen oder aber beispielsweise einen oder mehrere seitliche Anschläge an den Kanten der oberen Fläche, die verhindern, dass die Gegenkontaktflächen 32.1 32.2, 32.3 und 32.4 den Kontakt zu den Federkontakten 43, 44, 45 und 46 verlieren. Der Anschlag kann aber auch so ausgestaltet sein, dass der Gegenkontaktträger 32 und/oder 34 den Kontakt zum Kontaktträger 40 und 41 verliert. So werden die integrierten Schaltkreise oder elektrischen Komponenten 52 vor mechanischer Belastung geschützt und die Lebensdauer des medizinischen Implantats verlängert.

Die in den Figuren 1 bis 9 dargestellten medizinischen Implantate stellen nur besonders bevorzugte Ausführungsformen des Gegenstandes der vorliegenden Anmeldung dar. Weitere Ausführungen ergeben sich aus dem Umfang der Patentansprüche.

## Patentansprüche

1. Medizinisches Implantat mit einem Gehäuse, welches wenigstens zwei elektrische Komponenten (30, 50, 51,51.2) flüssigkeitsdicht umschließt, **dadurch gekennzeichnet, dass**
- wenigstens zwei elektrische Komponenten über einen Federkontaktblock (40, 41) elektrisch leitend verbunden sind,
- wobei wenigstens eine der elektrischen Komponenten wenigstens eine Gegenkontaktfläche (32, 34) aufweist, die als flaches, elektrisch leitendes Oberflächenelement einer Oberfläche der Komponente ausgebildet ist,
- wobei der Federkontaktblock (40, 41) wenigstens einen elektrisch isolierenden Kontaktträger (42) und wenigstens einen von diesem gehaltenen, leitfähigen, elastischen Federkontakt (43, 44, 45, 46) aufweist,
- der wenigstens zwei elektrisch leitende Kontaktierflächen (43.1, 43.2, 44.1, 44.2, 45.1, 45.2, 46.1, 46.2) aufweist, die elektrisch leitend miteinander verbunden sind, und
- der so ausgebildet ist, dass wenigstens eine der Kontaktierflächen (43.2, 44.2, 45.2, 46.2) unter Druck auf die Kontaktierflächen elastisch federnd ausgelenkt wird und eine definierte Feder-Rückstellkraft aufbaut,
- wobei eine elektrische Verbindung zwischen zwei elektrischen Komponenten durch den Federkontaktblock so hergestellt ist, dass die Kontaktierfläche des Federkontaktes auf wenigstens einer Gegenkontaktfläche (32.1, 32.2, 32.3, 32.4) wenigstens einer Komponente aufliegt und eine durch die Feder-Rückstellkraft des Federkontaktes erzeugte Anpresskraft auf die Gegenkontaktfläche ausübt.

2. Medizinisches Implantat nach Anspruch 1, **dadurch gekennzeichnet, dass** eine der elektrischen Komponenten eine Spannungsquelle (30) und eine weitere der elektrischen Komponenten ein elektrischer Verbraucher (50) ist, und beide über den Federkontaktblock (40) elektrisch leitend miteinander verbunden sind.

3. Medizinisches Implantat nach Anspruch 2, **dadurch gekennzeichnet, dass** sowohl die Spannungsquelle (30), als auch der elektrische Verbraucher (50) über jeweils zumindest eine mit der Kontaktierfläche (43.2, 44.2, 45.2, 46.2) des Federkontaktes (43, 44, 45, 46) kompatible Gegenkontaktfläche (32.1, 32.2, 32.3, 32.4) verfügt und eine elektrische Verbindung zwischen dem elektrischen Verbraucher (50) und der Spannungsquelle (30) sowohl auf Seiten der Spannungsquelle, als auch auf Seiten des elektrischen Verbrauchers, mit Hilfe der durch die Feder-Rückstellkraft des Federkontakts bewirkten Anpresskraft zwischen der Kontaktierfläche zumindest eines Federkontaktes und der Gegenkontaktfläche geschlossen ist.

4. Medizinisches Implantat nach Anspruch 2, **dadurch gekennzeichnet, dass** der elektrische Verbraucher (50) fest mit dem Federkontakt (43, 44, 45, 46) verbunden ist, und eine elektrische Verbindung zwischen dem elektrischen Verbraucher und der Spannungsquelle (30) mit Hilfe der durch die Feder-Rückstellkraft des Federkontaktes bewirkten Anpresskraft zwischen der Kontaktierfläche (43.2, 44.2, 45.2, 46.2) dieses Federkontaktes und zumindest einer Gegenkontaktfläche (32.1, 32.2, 32.3, 32.4) der Spannungsquelle hergestellt ist.

5. Medizinisches Implantat nach Anspruch 2, **dadurch gekennzeichnet, dass** die Spannungsquelle (30) fest mit dem Federkontakt (43, 44, 45, 46) verbunden ist und eine elektrische Verbindung zwischen dem elektrischen Verbraucher (50) und der Spannungsquelle mit Hilfe der durch die Feder-Rückstellkraft des Federkontaktes bewirkten Anpresskraft zwischen der Kontaktierfläche (43.2, 44.2, 45.2, 46.2) dieses Federkontaktes und zumindest einer Gegenkontaktfläche (32.1, 32.2, 32.3, 32.4) des elektrischen Verbrauchers hergestellt ist.

6. Medizinisches Implantat nach einem der Ansprüche 1 bis 5, **dadurch gekennzeichnet, dass** das flüssigkeitsdichte Gehäuse aus zwei Gehäuseteilen (20, 21) zusammengesetzt ist, wobei eines der Gehäuseteile (21) fest mit dem elektrischen Verbraucher (51) verbunden ist.

7. Medizinisches Implantat nach einem der Ansprüche 1 bis 6, **dadurch gekennzeichnet, dass** das flüssigkeitsdichte Gehäuse aus zwei Gehäuseteilen (20, 21) zusammengesetzt ist, wobei eines der Gehäuseteile (20) fest mit der Spannungsquelle (30) verbunden ist.

8. Medizinisches Implantat nach einem der Ansprüche 1 bis 7, **dadurch gekennzeichnet, dass** die Federkontakte (43, 44, 45, 46) aus einer gut leitfähigen Legierung, vorzugsweise aus einer kupferhaltigen Legierung, bestehen.

9. Medizinisches Implantat nach einem der Ansprüche 1 bis 8, **dadurch gekennzeichnet, dass** zumindest eine der Kontaktierflächen (43.2, 44.2, 45.2, 46.2) mit einem edlen Metall oder einer Legierung beschichtet ist, vorzugsweise mit Gold oder nickelhaltiger Legierung.

10. Medizinisches Implantat nach einem der Ansprüche 1 bis 9, **dadurch gekennzeichnet, dass** die Federkontakte (43, 44, 45, 46) einen im Wesentlichen flachen Querschnitt aufweisen.

11. Medizinisches Implantat nach Anspruch 10, **dadurch gekennzeichnet, dass** ein jeweiliger Federkontakt (43, 44, 45, 46) wenigstens eine Biegung in ausschließlich einer Ebene aufweist, welche orthogonal zu einer Oberfläche des Federkontaktes verläuft.

12. Medizinisches Implantat nach Anspruch 11, **dadurch gekennzeichnet, dass** die Biegung in einer Ebene verläuft, welche orthogonal zu einer Flachseite des Federkontaktes (43, 44, 45, 46) verläuft.

13. Medizinisches Implantat nach einem der Ansprüche 1 bis 12, **dadurch gekennzeichnet, dass** ein jeweiliger Federkontakt (43, 44, 45, 46) wenigstens eine Biegung mit einem Radius von weniger als 2 mm aufweist.

14. Medizinisches Implantat nach einem der Ansprüche 1 bis 13, **dadurch gekennzeichnet, dass** die Kontaktierfläche (43.2, 44.2, 45.2, 46.2) des Federkontaktes konvex in Richtung der Gegenkontaktfläche (32.1, 32.2, 32.3, 32.4) gewölbt ist.

15. Medizinisches Implantat nach einem der Ansprüche 1 bis 14, **dadurch gekennzeichnet, dass** der elektrische Verbraucher einen integrierten Schaltkreis umfasst.

16. Medizinisches Implantat nach einem der Ansprüche 1 bis 15, **dadurch gekennzeichnet, dass** der Kontaktträger (42) einen mechanischen Anschlag bildet, der Relativbewegungen senkrecht oder waagerecht zur oberen Fläche des Kontaktträgers verhindert.
